# EUROPEAN PATENT SPECIFICATION

(11) **EP 1 514 566 B1**
(45) Date of publication and mention of the grant of the patent: **06.08.2008**
(21) Application number: 03102725.3
(22) Date of filing: 09.09.2003
(51) Int. Cl.: A61M 5/32

(54) **Syringe including a retaining element with an integral annular rib**
Spritze mit einem eine integrale ringförmige Rippe aufweisenden Halteelement
Seringue comportant un élément de retenue avec une nervure annulaire integrée

(43) Date of publication of application: 16.03.2005
(73) Proprietor: Huang, Ping-Te, Ta-Li City, Taichung Hsien (TW)
(72) Inventor: Huang, Ping-Te, Ta-Li City, Taichung Hsien (TW)
(74) Representative: Zeitler - Volpert - Kandlbinder

(56) References cited:
- US-A- 5 968 019
- US-A1- 2003 060 775
- US-B1- 6 402 721
- US-B1- 6 423 033

## Description

This invention relates to a syringe, and more particularly to a syringe that includes a retaining element, which is formed with an integral annular rib, the annular rib serving as an O-ring.

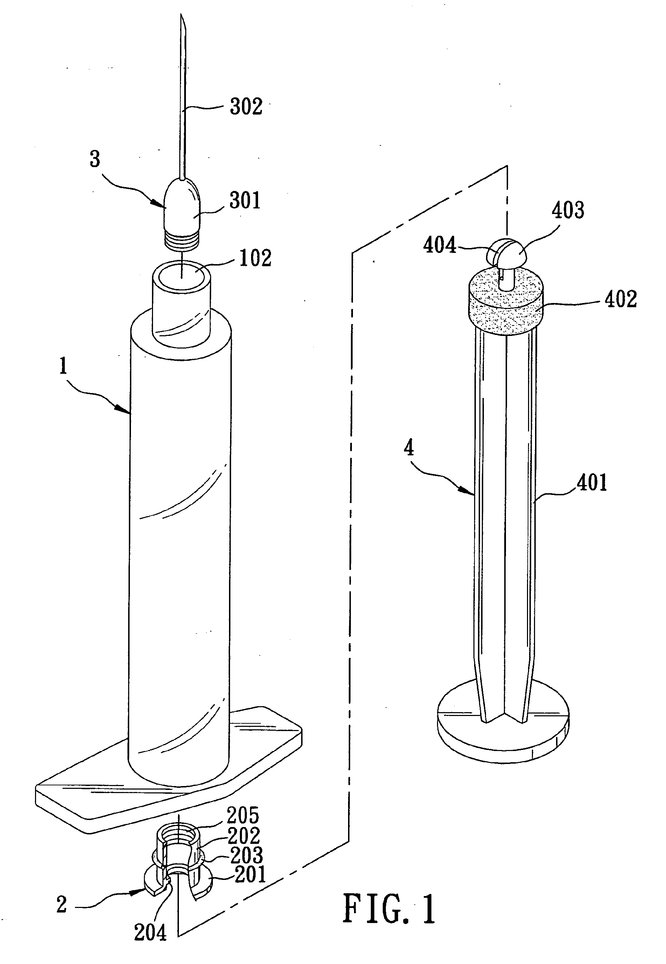

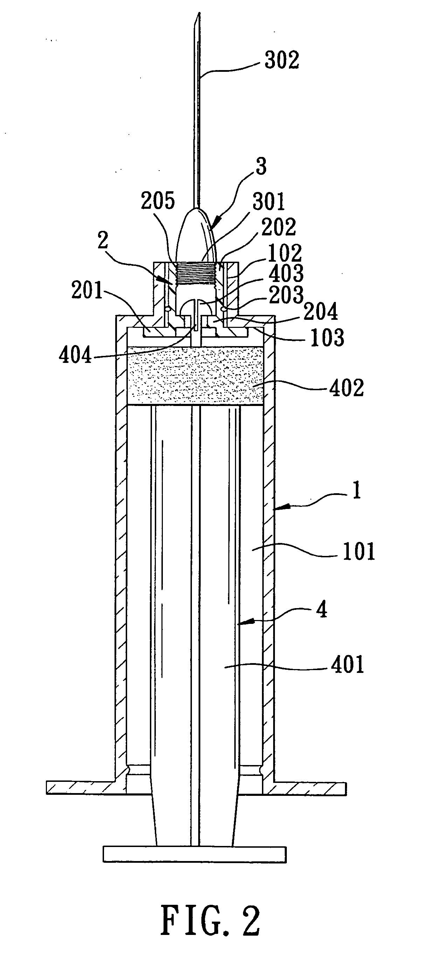

Referring to Figs. 1 and 2, a conventional syringe includes a syringe barrel 1, a retaining seat 2, a needle unit 3 connected threadably to the retaining seat 2, and a plunger 4. The barrel 1 has an interior chamber 101, a front end opening 102, and a shoulder 103. The retaining seat 2 is tubular, and includes an outward flange 201 extending radially and outwardly from a rear end of a cylindrical portion 202, an O-ring 203 sleeved on the cylindrical portion 202, and an inward flange 204 extending radially and inwardly from the cylindrical portion 202 and disposed immediately in front of the outward flange 201. The cylindrical portion 202 has an internally threaded front end 205. The needle unit 3 includes an externally threaded needle seat 301 engaging threadably the front end 205 of the cylindrical portion 202 of the retaining seat 2, and a needle body 302. The plunger 4 is movable within the barrel 1 between a front limit position and a rear limit position, and has an actuation rod portion 401, a piston 402 disposed movably and sealingly within the interior chamber 101 in the barrel 1, and a mushroom-shaped tongue 403 extending forwardly from the piston 402. When the plunger 4 is moved to the front limit position, the tongue 403 engages a hole defined by the inward flange 204 of the retaining seat 2 so as to permit synchronous movement of the retaining seat 2, the needle unit 3, and the plunger 4 when the plunger 4 moves back to the rear limit position, thereby retracting the needle body 32 into the barrel 1. The tongue 403 has a head that is formed with a slot 404 so as to permit the head to move into the needle seat 301 through the hole defined by the inward flange 103 and so as to prevent removal of the head from the needle seat 301 through the hole defined by the inward flange 103. As such, after use, the needle unit 3 can be brought into the barrel 1 by moving the plunger 4 from the front limit position to the rear limit position so as to prevent the needle body 32 from hurting the user. The aforesaid conventional syringe suffers from the following disadvantages:
1. The retaining seat 2 and the O-ring 203 are two separate members, which are manufactured by using two different machines, thereby increasing the manufacturing costs.
2. Because the size of the O-ring 203 is relatively small, it is difficult to sleeve the O-ring 203 manually around the retaining seat 2 so that the O-ring 203 is normally mounted on retaining seat 2 in an automated manner. However, the O-ring 203 may escape from its predetermined position during mechanical assembly in view of its elasticity. In the event that the escaped O-ring 203 moves into a space between two moving parts of a machine, the mounting operation may be interrupted for servicing of the machine.
3. During assembly, it is necessary to rotate the retaining seat 2 and the needle unit 3 relative to each other, thereby resulting in a troublesome process.

Document US 5 968 019 refers to a safety syringe including an injection head, a plunger and a barrel. The injection head has a needle cannula mounted to a needle seat, with the needle seat attachable to a needle seat connector. The inner side of the rear end of the needle seat connector has stoppers that correspond to protrusions on the coupling portion of the plunger. The plunger has a plunger shaft connected by a conical section to a coupling portion that has a rubber gasket. The plunger shaft can be snapped off at the conical section after use, so as to completely disable the syringe. The safety syringe has a barrel for receiving the head portion and the plunger such that a liquid-tight chamber is former to hold medicine or blood. The injection head is attached to the barrel through interlocking annular edges and grooves, and remains so attached during the withdrawal of fluid and during the injection phase. After the injection, the user simply rotates the plunger shaft, engages the stoppers of the needle seat connector with the protrusions of the plunger, and pulls the injection head onto the barrel of the syringe with the plunger. Once extracted, the plunger is broken off at the conical section, thereby protecting the needle cannula and preventing reuse.

Document US 6 423 033 B1 refers to a safety hypodermic syringe in which a pull handle is inserted into the plunger and adapted to pull the needle assembly backwards to the inside of the plunger in the barrel after the service of the safety hypodermic syringe. The pull handle has a middle neck through which the rear part of the pull handle can easily be separated from the front part of the pull handle by bending, enabling the separated rear part of the pull handle to be inserted into the plunger in the barrel and hook up with the front part of the pull handle.

According to this invention, a syringe includes the features defined in claim 1.

These and other features and advantages of this invention will become apparent in the following detailed description of the preferred embodiments of this invention, with reference to the accompanying drawings, in which:
Fig. 1 is an exploded perspective view of a conventional syringe;
Fig. 2 is a longitudinal sectional view of the conventional syringe;
Fig. 3 is an exploded sectional view of the first preferred embodiment of a syringe according to this invention;
Fig. 4 is a sectional view of a retaining seat unit of the first preferred embodiment, taken along Line 4-4 in Fig. 3;
Fig. 5 is a longitudinal sectional view of the first preferred embodiment;
Fig. 6 is a longitudinal sectional view of the first preferred embodiment, illustrating a front limit position of a plunger; and
Fig. 7 is a longitudinal sectional view of the first preferred embodiment, illustrating a rear limit position of the plunger, which is broken at a neck portion;

Before the present invention is described in greater detail in connection with the preferred embodiments, it should be noted that similar elements and structures are designated by like reference numerals throughout the entire disclosure.

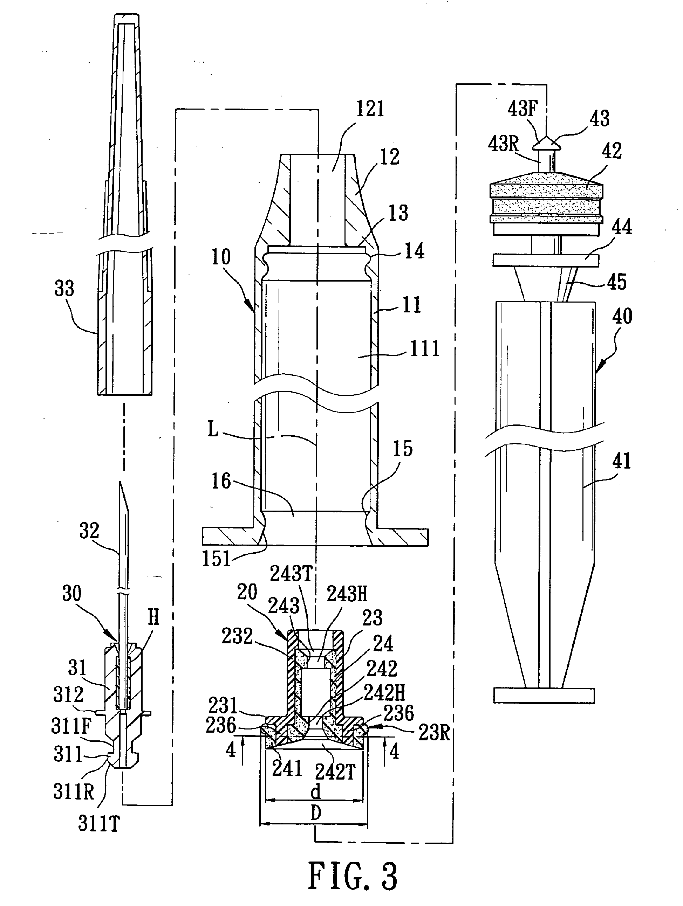

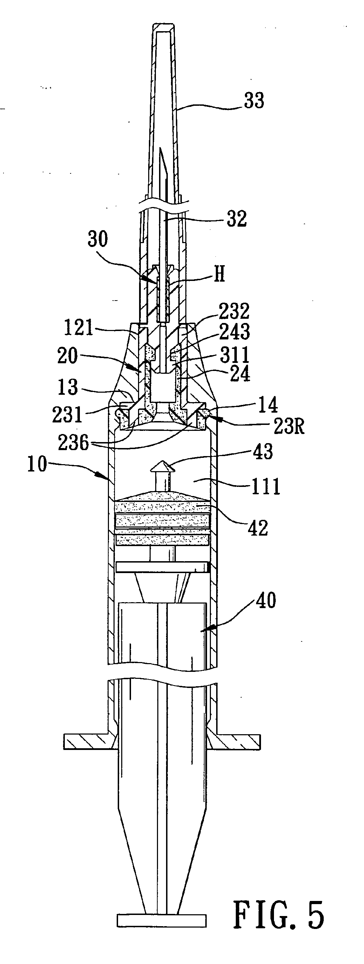

Referring to Figs. 3, 4, and 5, the preferred embodiment of a syringe according to this invention is shown to include a syringe barrel 10, a retaining seat unit 20 disposed in the barrel 10 , a needle unit 30 connected fixedly to the retaining seat unit 20, and a plunger 40 disposed movably within the barrel 10.

The barrel 10 has a central axis (L), a barrel body 11 with an interior chamber 111, a frontwardly converging front end portion 12 with a uniform-diameter front end opening 121 formed therethrough, a shoulder 13 formed between the front end opening 121 and the interior chamber 111, and an annular groove 13 formed in an inner surface of a front end of the barrel body 11. The barrel body 11 has a rear end that is formed with an integral inward flange 15 which extends radially and inwardly therefrom and which defines a rear clamping hole 16.

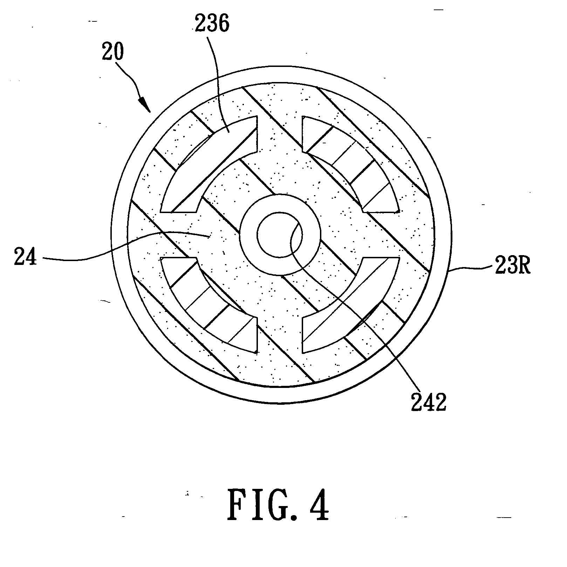

The retaining seat unit 20 is disposed in a front end of the barrel 10, and includes a unitary, rigid tubular main body 23 made of a plastic material and disposed within the barrel 10, and a unitary, tubular retaining element 24 made of rubber and fixed within the main body 23. The main body 23 has a rear end that is formed with an integral outward flange 231, a front cylindrical portion 232, and four projections 236 (see Fig. 4) extending rearwardly from the outward flange 231. The outward flange 231 of the main body 23 abuts against the shoulder 13 of the barrel 10. The retaining element 24 has a rear end that is formed with an outward flange 241 and a rear inward flange 242, and a front end, which is disposed within the main body 23, which extends into the front end opening 121 in the barrel 10, and which is formed with a front inward flange 243. The inward flanges 243, 242 define respectively front and rear clamping holes (243H, 242H), which are coaxial with the barrel 10. The retaining element 24 is molded on the main body 23 so that the outward flange 241 of the retaining element 24 is secured to the projections 236 of the main body 23. As such, the outward flange 241 of the retaining element 24 abuts against and is connected fixedly to the outward flange 231 of the main body 23. An annular rib (23R) is formed on an annular outer surface of the outward flange 241 of the retaining element 24, and has an outer diameter (D) that is greater than the outer diameter (d) of the outward flanges 231, 241 of the main body 23 and the retaining element 24, as shown in Fig. 3. That is to say, the retaining seat unit 20 has a maximum outer diameter at the annular rib (23R). The annular rib (23R) engages the annular groove 14 in the barrel 10 so as to anchor the retaining seat unit 20 within the barrel 10, and serves as an O-ring so as to establish a liquid-tight seal between the barrel 10 and the retaining seat unit 20.

The needle unit 30 includes a needle seat 31, a needle body 32 inserted into and fixed within the needle seat 31 by an adhesive material (H) , and a cover body 33 sleeved on the needle seat 31 and the needle body 32. The needle seat 31 has a mushroom-shaped tongue 311 at a rear end, and an outward flange 312 abutting against a rear end of the cover body 33. The tongue 311 has a uniform-diameter front tongue portion (311F) extending through the front clamping hole (243H), and a large-diameter rear tongue portion (311R) connected to a rear end of the front tongue portion (311F) and having a maximum diameter greater than the diameter of the front tongue portion (311F). The rear tongue portion 311R abuts against a rear end of the front inward flange 243 of the retaining seat unit 24 so as to retain the needle seat 31 on the retaining seat unit 20. The rear tongue portion (311R) of the tongue 311 of the needle seat 31 has a frusto-conical rear end (311T), and the front clamping hole (243H) in the retaining element 24 has a frusto-conical front end (243T) so as to guide the tongue 311 of the needle seat 31 to engage the front clamping hole (243H) in the retaining element 24.

The plunger 40 is disposed within the barrel 10, is located behind the retaining seat unit 20, and includes an actuation rod portion 41, a piston 42, a mushroom-shaped tongue 43, a circular abutment plate 44, and a neck portion 45 having two ends connected respectively and integrally with the actuation rod portion 41 and the abutment plate 44. The actuation rod portion 41 proj ects rearwardly from the barrel 10. The piston 42 is disposed movably and sealingly within the barrel 10, and is connected fixedly to and is disposed in front of the abutment plate 44. The tongue 43 is connected fixedly to and extends forwardly from the piston 42. The tongue 43 has a conical front head portion (43F) and a uniform-diameter rear neck portion (43R) that is connected to a rear end of the front head portion (43F). The front head portion (43F) has a maximum diameter greater than the diameter of the rear neck portion (43R), and a rear end surface that is perpendicular to the central axis (L) of the barrel 10. The rear clamping hole (242H) in the retaining element 242 has a frusto-conical rear end (242T) so as to guide the tongue 43 to engage the rear clamping hole (242H). The neck portion 45 has a diameter smaller than those of the abutment plate 44 and the actuation rod portion 41.

The inward flange 15 of the barrel 10 has an inclined rear end 151 (see Fig. 3) so that the piston 42 and the abutment plate 44 of the plunger 40 can be forced to move into the barrel 10.

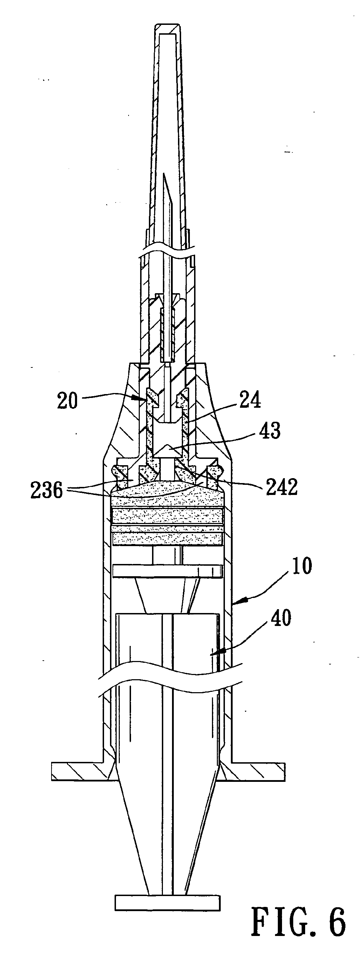

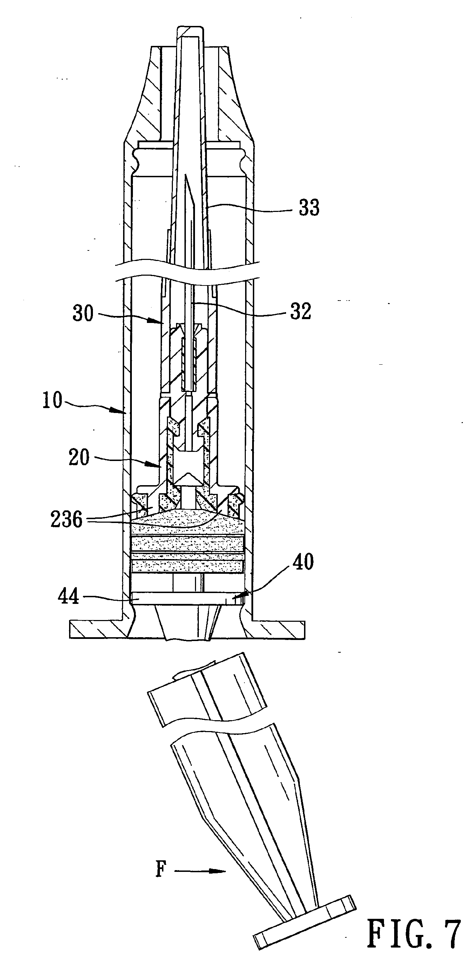

After use, the plunger 40 can be moved within the barrel 10 to a front limit position shown in Fig. 6, where the rear neck portion (43R) of the tongue 43 extends through the rear clamping hole (242H) and where the front head portion (43F) of the tongue 43 abuts against a front end of the inward flange 242 of the retaining element 24 so as to retain the retaining seat unit 20 on the tongue 43, thereby permitting synchronous movement of the plunger 40 and the retaining seat unit 20 when the plunger 40 moves rearward within the barrel 10 so as to retract the needle body 32 into the barrel 10. Subsequently, the plunger 40 is moved within the barrel 10 to a rear limit position shown in Fig. 7, where the abutment plate 44 abuts against a front end of the inward flange 15, where the needle body 32 is retracted into the barrel 10, and where a force can be applied to the actuation rod portion 41 in a transverse direction (F) relative to the plunger 40 so as to break the plunger 40 at the neck portion 45.

Some of the advantages of the syringe of this invention can be summarized as follows:
1. Because the annular rib (23R) is formed integrally with the retaining element 24, it is not necessary to provide an O-ring around the retaining seat unit 20. As such, the drawbacks associated with the presence of the O-ring in the conventional syringe are eliminated.
2. The needle unit 3 0 can be mounted easily on the retaining seat unit 20.

## Claims

1. A syringe including a syringe barrel (10), a retaining seat unit (20) disposed in a front end of the barrel (10) including a rigid tubular main body (23) disposed in the front end of the barrel (10), a needle unit (30) connected fixedly to the retaining seat unit (20), and a plunger (40) disposed movably within the barrel (10) and behind the retaining seat unit (20), wherein the retaining seat unit (20) further includes a tubular retaining element (24) fixed within the main body (23), the retaining element (24) having an annular outer surface that is formed with an annular rib (23R), and an assembly of the main body (23) and the retaining element (24) having a maximum outer diameter at the annular rib (23R) such that the annular rib (23R) can contact an inner surface of the barrel (10), thereby establishing a liquid-tight seal between the barrel (10) and the assembly of the main body (23) and the retaining element (24),
wherein
the barrel (10) has a front end opening (121); the retaining element (24) having a front end that extends into the opening (121) in the barrel (10) and that is formed with an integral front inward flange (243) which extends radially and inwardly therefrom and which defines a front clamping hole (243H); and the needle unit (30) includes a needle seat (31) and a needle body (32) connected fixedly to the needle seat (31), the needle seat (31) being formed with an integral mushroom-shaped tongue (311) which has a uniform-diameter front tongue portion (311F) extending through the front clamping hole (243H) in the retaining seat unit (20), and a rear tongue portion (311 R) connected to a rear end of the front tongue portion (311 F) and having a maximum diameter greater than the diameter of the front tongue portion (311 F), the rear tongue portion (311 R) abutting against a rear end of the front inward flange (243) of the retaining seat unit (24) so as to retain the needle seat (31) on the retaining seat unit (20);
the rear tongue portion (311 R) of the tongue (311) of the needle seat (31) has a frusto-conical rear end (311T), and the front clamping hole (243H) in the retaining element (24) has a frusto-conical front end (243T) so as to guide the tongue (311) of the needle seat (31) to engage the front clamping hole (243H) in the retaining element (24);
the main body (23) of the retaining seat unit (20) has a rear end that is formed with an outward flange (231) extending radially and outwardly therefrom, and the retaining element (24) of the retaining seat unit (20) has a rear end that is formed with an outward flange (241) which abuts against and which is secured to the outward flange (231) of the main body (23), the annular rib (23R) being formed on the outward flange (241) of the retaining element (24);
the outward flange (231) of the main body (23) is formed with a plurality of projections (236) extending rearwardly therefrom, the outward flange (241) of the retaining element (24) being molded on the projections (236) so as to connect the retaining element (24) fixedly to the main body (23);
the retaining element (24) of the retaining seat unit (20) has a rear end that is formed with an integral inward flange (242) that defines a rear clamping hole (242H), the plunger (40) having a front end that is formed with an integral mushroom-shaped tongue (43) which has a front head portion (43F) and a uniform-diameter rear neck portion (43R) that is connected to a rear end of the front head portion (43F), the front head portion (43F) having a maximum diameter greater than the diameter of the rear neck portion (43R), the plunger (40) being movable within the barrel (10) between a rear limit position and a front limit position, where the rear neck portion (43R) of the tongue (43) extends through the rear clamping hole (242H) and where the front head portion (43F) of the tongue (43) abuts against a front end of the inward flange (242) of the retaining element (24) so as to retain the retaining seat unit (20) on the tongue (43), thereby permitting synchronous movement of the plunger (4 0) and the retaining seat unit (20) when the plunger (40) moves rearward within the barrel (10) so as to retract the needle body (32) into the barrel (10);
the front head portion (43F) of the tongue (43) of the plunger (40) is conical, and the rear clamping hole (242H) in the retaining element (242) has a frusto-conical rear end (242T) so as to guide the tongue (43) to engage the rear clamping hole (242H);
the barrel (10) has a rear end that is formed with an integral inward flange (15) that defines a rear end opening (16) of the barrel (10), the plunger (40) further having an actuation rod portion (41) projecting rearwardly from the barrel (10); a piston (42) disposed between and connected fixedly to the tongue (43) and the actuation rod portion (41) and disposed movably and sealingly within the barrel (10); an abutment plate (44) disposed between and connected fixedly to the piston (42) and the actuation rod portion (41), the abutment plate (44) abutting against a front end of the inward flange (15) of the barrel (10) when the plunger (40) is disposed at the rear limit position; and a neck portion (45) having two ends connected respectively and fixedly to the abutment plate (44) and the actuation rod portion (41), and a diameter smaller than those of the abutment plate (44) and the actuation rod portion (41), the neck portion (45) extending through the rear end opening (16) in the barrel (10) when the plunger (40) is disposed at the rear limit position so as to permit the plunger (40) to be broken at the neck portion (45) by applying a force to the actuation rod portion (41) in a transverse direction relative to the plunger (40).

2. The syringe as claimed in claim 1, **characterized in that** the inner surface of the barrel (10) is formed with an annular groove (14), the annular rib (23R) of the retaining element (24) engaging the annular groove (14) so as to anchor the retaining seat unit (20) within the barrel (10).

## Patentansprüche

1. Injektionsspritze, bestehend aus einem Zylinder (10); einer Haltesitzanordnung (20), die an einem Vorderende dess Zylinders (10) angeordnet ist und einen am Vorderende des Zylinders (10) angeordneten rigiden rohrförmigen Hauptkörper (23) aufweist; einer Nadelanordnung (30), die mit der Haltesitzanordnung (20) fest verbunden ist; und einer Kolbenstange (40), die beweglich innerhalb des Zylinders (10) und zwar hinter der Haltesitzanordnung (20) angeordnet ist, wobei die Haltesitzanordnung (20) ferner ein rohrförmiges Befestigungselement (24) aufweist, das innerhalb des rohrförmigen Hauptkörpers (23) fest verbunden ist und eine ringförmige Außenoberfläche aufweist, die mit einer ringförmigen Rippe (23R) ausgestaltet ist, wobei ein maximaler Außendurchmesser an der ringförmigen Rippe (23R) derart angeordnet ist, dass die ringförmige Rippe (23R) mit einer Innenoberfläche des Zylinders (10) in Kontakt kommen kann, wodurch eine flüssigkeitsdichte Dichtung zwischen dem Zylinder (10) und der Anordnung des Hauptkörpers (23) und dem Befestigungselement (24) ausgestaltet ist,
**dadurch gekennzeichnet,**
**dass** der Zylinder (10) eine Vorderende-Öffnung (121) aufweist; dass das rohrförmige Befestigungselement (24) ein Vorderende besitzt, das sich in die Öffnung (121) im Zylinder (10) erstreckt und mit einem integralen vorderen Innenflansch (243) ausgestaltet, der sich radial, von demselben innenwärts erstreckt und grenzt ein vorderes Spannloch (243H) um; dass die Nadelanordnung (30) einen Nadelträger (31) und einen Nadelkörper (32) umfasst, der mit dem Nadelträger (31) fest verbunden ist, wobei der Nadelträger (31) mit einer integralen pilzförmigen Zunge (311) ausgestaltet ist, die ein Uniform-Durchmesser-Vorderzungenteil (311F) aufweist, das sich durch das vordere Spannloch (243H) in die Haltesitzanordnung (20) erstreckt, wobei ein Hinterzungenteil (311 R) mit einem Hinterende des Vorderzungenteils (311 F) verbunden ist und einen maximalen Durchmesser besitzt, der größer ist als der Durchmesser des Vorderzungenteils (311 F), wobei das Hinterzungenteil (311 R) an ein Hinterende des vorderen Innenflanschs (243) des Befestigungselements (24) angrenzt, um die Nadelanordnung (31) an der Haltesitzanordnung (20) zu befestigen;
**dass** das hintere Zungenteil (311 R) der Zunge (311) des Nadelträgers (31) ein frusto-konisches Hinterende (311T) aufweist, und das vordere Spannloch (243H) im Befestigungselement (24) ein frusto-konisches Vorderende (243T) aufweist, um die Zunge (311) des Nadelträgers (31) zu führen und so das vordere Spannloch (243H) mit dem Befestigungselement (24) in Rastverbindung zu bringen;
**dass** der Hauptkörper (23) der Haltesitzanordnung (20) ein Hinterende besitzt, das mit einem Außenflansch (231) der sich radial, von demselben außenwärts erstreckt, und das Befestigungselement (24) der Haltesitzanordnung (20) ein Hinerende besitzt, das mit einem Außenflansch (241) ausgestaltet ist, der an dem Außenflansch (231) angrenzt und am Außenflansch (231) des Hauptkörpers (23) befestigt ist, wobei die ringförmige Rippe (23R) am Außenflansch (241) des Befestigungselements (24) ausgestaltet ist;
**dass** der Außenflansch (231) des Hauptkörpers (23) mit einer Vielzahl von Vorsprüngen (236) ausgestaltet ist, die sich rückwärts und von demselben erstreckt, wobei der Außenflansch (241) des Befestigungselements (24) an den Vorsprüngen (236) angeformt ist, um das Befestigungselement (24) am Hauptkörper (23) fest zu verbinden;
**dass** das Befestigungselement (24) der Haltesitzanordnung (20) ein Hinterende besitzt, das mit einem integralen Innenflansch (242) ausgestaltet ist, der an einem hinteren Spannloch (242H) angrenzt, wobei die Kolbenstange (40) ein Vorderende aufweist, das mit einer integralen pilzförmigen Zunge (43) ausgestaltet ist, die mit einem vorderen Kopfteil (43F) und einem Uniform-Durchmesser-Hintenhalsteil (43R) versehen ist, das mit einem Hinterende des vorderen Kopfteils (43F) verbunden ist, das einen maximalen Durchmesser besitzt, der größer ist als der Durchmesser des Hinterzungenteils (43R), wobei die Kolbenstange (40) innerhalb des Zylinders (10) zwischen einer hinteren Limitposition und einer vorderen Limitposition beweglich ist, wo das hintere Halsteil(43R) der Zunge (43) sich durch das hintere Spannloch (242H) und wo das vordere Kopfteil (43F) der Zunge (43) an einem Vorderende des Innenflansches (242) des Befestigungselements (24) angrenzt, um die Haltesitzanordnung (20) an der Zunge (43) zu befestigen, wobei synchrone Bewegungen der Kolbenstange (40) und der Haltesitzanordnung (20) erlaubt werden, wenn sich die Kolbenstange (40) rückwärts innerhalb des Zylinders (10) bewegt, um den Nadelkörper (32) in den Zylinder (10) einzuziehen ;
**dass** das vordere Kopfteil (43F) der Zunge (43) der Kolbenstange (40) konisch ist, und das hintere Spannloch (242H) im Befestigungselement (24) ein frusto-konisches Hinterende (242T) aufweist, um die Zunge (43) so zu führen, dass die Zunge (43) in das hintere Spannloch (242H) einrastet;
**dass** der Zylinder (10) ein Hinterende aufweist, das mit einem integralen Innenflansch (15) ausgestaltet ist, der an einer Hinterende-Öffnung (16) des Zylinders (10) angrenzt, wobei die Kolbenstange (40) ferner einen Auslösestab (41) aufweist, der sich vom Zylinder (10) rückwärts vorspringt; dass ein Kolben (42) zwischen der Zunge (43) und dem Auslösestab (41) angeordnet und mit denselben fest verbunden sind und abgedichtet innerhalb des Zylinders (10) beweglich angeordnet ist; dass eine Brückenplatte (44) zwischen dem Kolben (42) und dem Auslösestab (41) angeordnet und mit denselben fest verbunden ist, wobei die Brückenplatte (44) an einem Vorderende des Innenflansches (15) des Zylinders (10) angrenzt, wenn die Kolbenstange (40) an der hinteren Limitposition angeordnet wird; und dass ein Halsteil (45) zwei Enden aufweist, die jeweils mit der Brückenplatte (44) und dem Auslösestab (41) verbunden, wobei ein Durchmesser kleiner als die Durchmesser der Brückenplatte (44) und des Auslösestabs (41) ist, wobei das Halsteil (45) sich durch die Hinterende-Öffnung (16) in den Zylinder (10) erstreckt, wenn die Kolbenstange (40) an der hinteren Limitposition angeordnet wird, um durch Ausübung einer Kraft auf den Auslösestab (41) in eine relativ zur Kolbenstange (40) querlaufende Richtunge die Kolbenstange (40) zu brechen.

2. Injektionsspritze nach Anspruch 1**,**
**dadurch gekennzeichnet,**
**dass** die Innenoberfläche des Zylinders (10) mit einer ringförmigen Nut (14) ausgestaltet ist, wobei die ringförmige Rippe (23R) des rohrförmigen Befestigungselements (24) in die ringförmige Nut (14) einrastet, um die Haltesitzanordnung (20) innerhalb des Zylinders (10) zu verankern.

## Revendications

1. Une seringue contient un cylindre de seringue(10), un support de rétention (20) disposée dans la partie avant du cylindre (10) contenant un corps principal tubulaire rigide(23) disposé dans la partie avant du cylindre (10), une unité d'aiguille(30) est connectée fermement au support de rétention (20), et un piston (40) disposé en mode mobile dans le cylindre (10) et derrière le support de rétention (20), dans lequel le support de rétention (20) comprend en outre un élément de rétention tubulaire (24) qui est relié fermement au corps principal (23), l'élément de rétention (24) ayant une surface externe annulaire qui est formée par une structure annulaire (23R), et un diamètre extérieur maximal à la structure annulaire (23R) de sorte que la structure annulaire (23R) puisse contacter une surface intérieure du cylindre (10), créant ainsi d'un organe étanche-liquide entre le cylindre (10) et l'assemblàge du corps principal (23) et l'élément de rétention(24).
**Caractérisé aux points suivants:**
Le cylindre(10) ayant un orifice frontal au bout(121), l'élément de rétention(24) ayant une extrémité frontale qui se prolonge dans l'orifice(121) dans le cylindre (10), et qui est formé avec une bride intérieure frontale intégrale (243) qui s'étend ainsi radialement et intérieurement et qui définit un trou de serrage avant (243H), et l'unité d'aiguille (30), y compris un porte-aiguille (31) et un corps de l'aiguille (32) connecté fermement au porte-aiguille (31), le porte-aiguille (31) formée avec languette sous forme de champignon (311) qui a une partie avant de la languette à diamètre uniforme(311F) étendant à travers le trou de serrage avant (243H) dans le support de rétention (20), et une partie arrière de la languette (311 R) reliée à l'extrémité arrière de la partie arrière de la languette(311 F) et ayant un diamètre maximal supérieur au diamètre de la partie avant de la languette (311 F), la partie arrière de la languette (311 R) adjacent à l'encontre d'une extrémité arrière de la bride intérieure avant(243) de l'unité de rétention(24) de façon à conserver le porte-aiguille (31) sur le support de rétention (20);
La partie de languette arrière(311 R) de la languette (311) du porte-aiguille (31) a une extrémité arrière tronconique (311T), et le trou de serrage avant (243H) dans l'élément de rétention(24) a une extrémité avant tronconique(243T), afin de guider la languette (311) du porte-aiguille (31) d'engager le trou de serrage avant (243H) dans l'élément de rétention(24);
Le corps principal (23) du support de rétention (20) a une extrémité arrière qui est formée avec une bride extérieure (231) s'étendant ainsi radialement et vers l'extérieur, et l'élément de rétention(24) du support de rétention (20) a une extrémité arrière qui est formée avec une bride extérieure(241) qui jouxte à l'encontre et qui est fixée à la bride extérieure (231) du corps principal(23), la structure annulaire (23R) a été formée sur la bride extérieure (241) de l'élément de rétention (24);
La bride extérieure(231) du corps principal(23) est formé avec la plupart de projections (236) étendant vers l'arrière, la bride extérieure(241) de l'élément de rétention (24) étant moulée sur les projections (236), afin de connecter l'élément de rétention(24) fermement au corps principal (23);
L'élément de rétention(24) du support de rétention(20) a une extrémité arrière qui est formée avec une bride intérieure intégrale (242) qui définit un trou de serrage arrière (242H), le piston (40) ayant une partie avant qui est formée avec une languette sous forme de champignon (43) qui dispose d'une tête frontale (43F) et une partie de collet arrière à diamètre uniforme (43R), qui est reliée à une extrémité arrière de la tête avant (43F), la partie de tête frontale(43F) ayant un diamètre maximal supérieur au diamètre de la partie de collet arrière (43R),Le piston (40) étant mobile dans le cylindre (10) entre une position limite arrière et une position limite frontale, où la partie de collet arrière(43R) de la languette (43) s'étend à travers le trou de serrage arrière (243H), et où la tête avant(43F) de la languette (43) jouxte contre une extrémité frontale de la bride intérieure(242) de l'élément de rétention (24) de façon à conserver le support de rétention (20) sur la languette (43), ce qui permet le mouvement synchrone du piston (40) et du support de rétention (20), lorsque le piston (40) se déplace vers l'arrière dans le cylindre(1 0) de manière à rentrer le corps de l'aiguille (32) dans le cylindre (10);
La tête frontale (43F) de la languette (43) du piston (40) est conique, et le trou de serrage arrière (242H) dans l'élément de rétention (242) a une extrémité tronconique arrière (242T), de manière à guider la languette (43) à engager le trou de serrage arrière (242H);
Le cylindre(1 0) a une extrémité arrière qui est formée avec une bride intérieure intégrale (15), qui définit un orifice à l'extrémité arrière(16) du cylindre(10), le piston (40) a autrement une partie de tige d'activation (41) projetant à partir du cylindre vers l'arrière (10).Un piston (42) et relié fermement à la languette (43) et disposé entre la languette et la tige d'activation (41) et disposé au mode mobile et étanche dans le cylindre (10).Une plaque de butée (44) reliée fermement au piston (42) et disposé entre le piston et la tige d'activation(41), la plaque de butée (44) adjacent à l'encontre d'une extrémité frontale de la bride intérieure (15) du cylindre (10) quand le piston (40) est disposé à la position limite arrière, et une partie de collet (45) ayant deux extrémités reliées respectivement fermement à la plaque de butée (44) et la tige d'activation (41), et d'un diamètre plus petit que ceci de la plaque butée (44) et la tige d'activation (41), le collet (45) s'étend par l'intermédiaire de l'orifice située à l'extrémité arrière(16) dans le cylindre (10) lorsque le piston (40) est disposé à la position limite arrière de manière à permettre le piston (40) à être brisé à la partie de collet(45) en appliquant une force sur la tige d'activation (41) dans un sens transversal par rapport au piston (40).

2. Selon la demande 1, la seringue est **caractérisée en ce que** la surface intérieure du cylindre (10) est formée avec une rainure annulaire (14), la structure annulaire(23R) de l'élément de rétention (24) engageant la rainure annulaire (14) de manière à attacher le support de rétention(20) au sein du cylindre (10).
